# EUROPEAN PATENT SPECIFICATION

(11) **EP 1 232 397 B1**
(45) Date of publication and mention of the grant of the patent: **17.11.2004**
(21) Application number: 00953588.1
(22) Date of filing: 21.08.2000
(51) Int. Cl.: G01N 33/74, G01N 33/543

(54) **METHOD TO DETERMINE PROGESTERONE IN RAW MILK**
VERFAHREN ZUM NACHWEIS VON PROGESTERON IN ROHMILCH
PROCEDE POUR DETERMINER LA TENEUR EN PROGESTERONE DE LAIT CRU

(30) Priority: 19.08.1999 NL 1012859
(43) Date of publication of application: 21.08.2002
(73) Proprietor: N.V. Nederlandsche Apparatenfabriek NEDAP, 7141 DC Groenlo (NL)
(72) Inventor: ROOSENSCHOON, Pieter, Lieuwe, NL-7104 AC Groenlo (NL); VERSTEGE, Albertino, Bernardo, Maria, NL-7121 HM Aalten (NL)
(74) Representative: Prins, Adrianus Willem, Mr. Ir.
(86) International application number: PCT/NL2000/000580
(87) International publication number: WO 2001/014887

(56) References cited:
- R.M. PEMBERTON ET AL.: "Development of a sensitive, selective electrochemical immunoassay for progesterone in cow's milk based on a disposable screen-printed amperometric biosensor " ELECTROCHIMICA ACTA, vol. 43, no. 23, 1998, pages 3537-3574, XP004130864 Barking UK
- CHEMICAL ABSTRACTS, vol. 130, no. 11, 15 March 1999 (1999-03-15) Columbus, Ohio, US; abstract no. 134247, XP002137666 & R. CLAYCOMB ET AL.: "Biosensor for online measurement of bovine" BIOSENSORS AND BIOELECTRONICS , vol. 13, no. 11, 1998, pages 1173-1180, Denver CO USA

## Description

This invention relates to a method and system for determining the amount of progesterone in raw milk. Such a method is known from the article RM PEMBERTON et al. (1998). Development of a sensitive, selective electrochemical immunoassay for progesterone in cow's milk based on a disposable screen-printed amperometric biosensor. ELECTROCHIMICA ACTA 43 (23), 3567-3574.

In dairy farming, it is of importance to know the estrous/heat cycle of a cow. On the basis of this cycle, the farmer can determine at what time the cow is in heat and what the subsequent time of insemination should be.

The time of insemination is difficult to determine. Accordingly, it happens regularly that the artificial insemination occurs too early or too late, so that fertilisation does not take place. This is partly due to the fact that the cycle of each individual cow is different. It is therefore desired to use an objective method of determining the time of artificial insemination.

An important parameter for determining the time of insemination is the progesterone concentration in the milk the cow produces. When the time at which the cow can be inseminated approaches, the progesterone concentration in the milk falls below 4 ng/ml. After the insemination the progesterone concentration rises above 20 ng/ml and when a fertilisation has taken place, the progesterone concentration will remain above 20 ng/ml. When the progesterone concentration decreases again, a new artificial insemination can take place. As each individual cow has a different cycle, it is desirable to be able to follow the cycle of each cow separately. To more accurately determine the time of insemination, it is not sufficient to know when the progesterone concentration is below or above the lower or upper limit. Especially reliable information about the concentration range between 0 and 7 ng/ml of progesterone is of importance. Most preferably, the farmer wants to make use of a method for progesterone determination which is automated to a high degree, has a high reliability and has a low cost price per sample.

It is known that the farmer determines the time of insemination on the basis of the condition of the cow or by utilising field analysis methods for determining the progesterone concentration in raw milk.

The condition of the cow can be determined inter alia by means of visual observation of the animal behaviour and/or an activity measurement. For this purpose, the cow has a step counter with which the number of steps can be counted which the animal makes during a defined time interval. This requires the farmer to observe his cows at least two or three times a day for 15 to 30 minutes. In addition, the farmer also looks at the behaviour of the cow. When the cow is in heat, the cow regularly mounts other cows and is mounted herself.

In addition to this century-old method, nowadays use is made of newly developed field analysis methods. To be considered are, for instance, dipstick tests, whereby the farmer takes a sample of milk from the cow (this is a manual operation and is not automated) and holds a test strip in the milk. After a minimum incubation period in which the progesterone in the milk reacts with the reagents in the test strip, it can be read what the concentration of progesterone in the milk under test is. In most cases, this reading is done visually, but in some cases the reading may be objectified by utilising a photometer. The accuracy of the analytic assay is not very high, so that a pronouncement can be made only on whether the concentration is above or below a set limit.

On the basis of the concentration, not only the estrous status can be checked, but also an early gestation diagnosis can be performed. Furthermore, it can be established whether the animal possibly has a defect of the reproductive apparatus.

For the farmer, it would be ideal if the determination of the concentration of progesterone in raw milk could be automated to a large extent. The farmer then has his hands free for carrying out other activities and, what is more, the farmer is not trained to perform an analytic assay.

In addition, it would be desirable to be able to determine the progesterone concentration with a high accuracy through the entire range between 0 and 40 ng/ml. This gives the farmer more information about the cycle of the individual cow and he can determine the time of insemination better for each cow. This will result in a reduction of the number of failed inseminations per cow, so that the productivity per cow will rise.

In a known method for measuring the amount of progesterone in milk with the aid of a biosensor, use is made of a screen printed carbonelectrode which is brought into contact with the milk in which for the measurement E(Enzyme) labelled progesterone is added. The sensor is partly provided with a biological coating. In use, an immunochemical interaction takes place between the progesterone (from the milk and the labelled progesterone) and this coating. During analysing a competition takes place between both types of progesterone. After incubation of the milk with the sensor, the surface is washed and an amount of substrate is provided to the enzyme. The substrate is transformed into an electrochemical active material after which a signal is induced. This occurs on a part of a surface of the sensor which is not provided with the coating. A known method involves therefore an electrochemical measurement.

Because the milk contacts both the coated as well as the non-coated part of the sensor, non-specific interactions take place which may have a negative influence on the reproducibility of the sensor. Furthermore three biochemical interactions are needed for obtaining a measurement signal wherein each interaction involves errors, so that the accuracy of the measurement is relatively low.

The object of the invention is to provide a solution to the above-mentioned disadvantages as well as to meet the wishes on the part of the farmer. In addition, the invention also has good utility for detecting status and/or defects in fertility.

The method according to the invention is accordingly characterised in that weighing of the plate is measured for determining the amount of progesterone.

The measurement of the weighing of the plate can be measured in a manner known per se. Because prior to the measurement of the weighing only one interaction has taken place, whereby the measurement of the weighing can also be carried out very accurate, the determination of the amount of progesterone can be carried out very accurate as well. Furthermore, the biosensor may be cleaned for re-use.

In particular, it holds that the biosensor is connected with a computer for the on-line determination of the amount of progesterone in milk. In this way, it is possible to condition the concentration determination of progesterone in raw milk to a high degree.

Preferably, it holds that further the identity of the animal is determined, the amount of progesterone is determined from raw milk which has been produced by the cow a plurality of times spread in time, while the identity of the animal, together with the determined amounts of progesterone and information about the times at which these amounts were determined and/or the milk was produced, are stored in a database. In this way, the course of the concentration of progesterone in raw milk, spread in time, can be determined per animal, be stored and be processed further. On the basis of the time-dependent concentration curve, it can be accurately determined when the cow is in heat, whether the cow is in-calf, or whether the cow possibly has a defect of its reproductive apparatus. In particular, it holds that the amount of progesterone in the milk is determined during milking. If the cow is milked with the aid of a milking robot plant, the biosensor can, if desired, be placed in the path along which the raw milk flows through the milking robot plant.

The invention also relates to a system comprising a computer and a biosensor connected thereto, which system is arranged for carrying out the method according to the invention.

The invention will presently be further elucidated with reference to the drawing. In the drawing:
Fig. 1 schematically shows a system according to the invention for carrying out a method according to the invention;
Fig. 2 shows the use according to the invention of a first type of biosensor;
Fig. 3 shows the alternative use according to the invention of a second type; and
Fig. 4 shows the curve in time of the concentration of progesterone in raw milk of a cow.

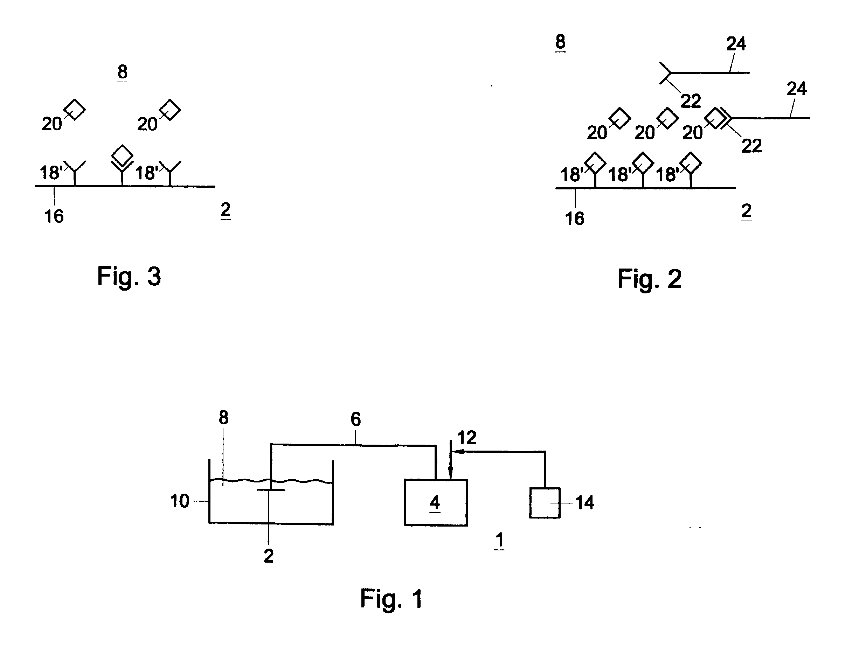

In Fig. 1 reference numeral 1 designates a system for carrying out a method according to the invention. The system comprises a biosensor 2, a computer 4, and a line 6 connecting the biosensor 2 with the computer 4.

For determining the amount of progesterone in the raw milk, the biosensor 2 is contacted with the raw milk 8 of which, in this case, the progesterone concentration is to be determined. The raw milk 8 in this example is contained in a container 10 which has many embodiments, as will be discussed hereinafter.

The raw milk 8 can consist, for instance, of a sample of the raw milk which is produced by the cow when being milked. This sample can then be introduced into the container 10.

The biosensor 2 is of a type known per se and is arranged to determine the concentration of progesterone in the raw milk 8. The biosensor 2 generates on line 6 a signal that represents the measured concentration of progesterone in the raw milk. In this example, this concentration is stored in a database of the computer 4. In this example, via an input 12 of the computer, also the identity of the animal from which the raw milk originates is stored in the database. The identity of the animal can be determined in a manner known per se. To be considered here are, for instance, determining the identity on the basis of a neck responder, an earmark, an implant and/or a bolus, etc. The system can be provided, for instance, with an identification device 14, known per se, which establishes the identity of the animal and feeds it accordingly to the input 12 of the computer 4.

In particular, it holds that in addition to the identity of the animal, the amount of progesterone is determined of raw milk which has been produced by the animal a plurality of times spread in time. The identity of the animal is then stored in the database together with the determined amounts of progesterone as well as information about the times at which the amounts were determined and/or the milk was produced.

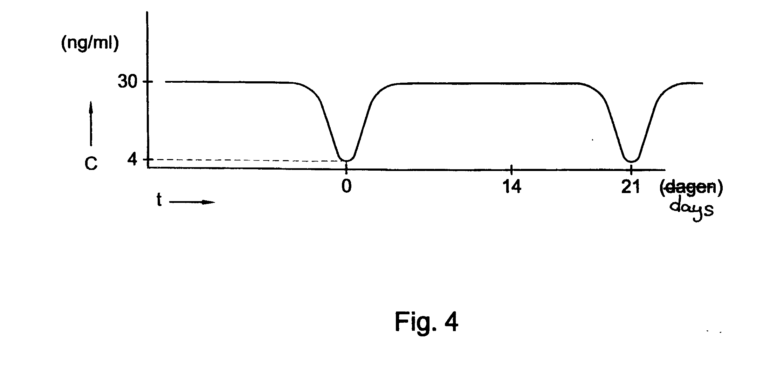

In this way, an insight is obtained into the course of the concentration in time, see also Fig. 4, where the horizontal axis plots time in days and the vertical axis plots the concentration in nanograms per milliliter. The time at which the animal is in heat is denoted by day zero and coincides with a dip in the concentration curve in time of the amount of progesterone in the raw milk. Twenty-one days later the animal proves to be in heat again. By presently signalling the time at which the concentration starts to decrease from a value of, for instance, 40 ng/ml to a value of aroun 4 ng/ml, the time of estrus can be accurately determined. When the concentration does not decrease roundabout the twenty-sixth day, the animal is either gestating or the animal possibly has a defect of its reproductive apparatus. Preferably, it holds that for about two weeks (fourteen days) after a change in the determined amount of progesterone in time has reached a minimum, the progesterone content of the milk of the cow is not determined. As appears from Fig. 4, in general only after the fourteenth day, when the animal is not gestating and/or does not have a defect of its reproductive apparatus, will the concentration decrease again. Only after fourteen days, therefore, is it of interest again to start monitoring the change over time of the amount of progesterone in the milk. After the fourteenth day, the concentration of progesterone in the raw milk of the animal can be determined, for instance, every two or three days.

Preferably, the amount of progesterone is determined during milking with the aid of a milking robot. The biosensor can then be positioned at a suitable position in a path along which the raw milk of the cow flows through the milking robot. To be considered here are, for instance, the milk line and/or the milk claw. In that case, therefore, the milk claw or the milk line constitutes the container 10 in Fig. 1. It is also possible, however, that in the milking robot in an automatic manner a sample is taken of the raw milk which flows along the milk flow path of the milking robot. This sample is then placed in a container 10 separate from the milk flow path. This is useful in particular when a biosensor is used that consists of a plate on which a coating of progesterone bodies is provided, as will be further elucidated hereinafter.

In that case, as shown in Fig. 2, the biosensor 2 can be provided with a plate 16 on which a coating is applied which comprises progesterone bodies 18. Also designated in Fig. 2 is the raw milk 8 which comprises progesterone 20. Further, to the sample of raw milk shown in Fig. 2 an amount of progesterone antibodies 22 has been added. In Fig. 2 the long tail 24 of the antibodies 22 is schematically shown.

If the concentration of antibodies 22 in the raw milk 8 is gradually raised in a manner known per se, the antibodies 22 will primarily bind to the progesterone bodies 20. As soon as more antibodies 22 have been supplied to the raw milk than there are progesterone bodies present in the raw milk, the antibodies 22 will also start to bind to the progesterone bodies 18 of the plate 16. The result is that the mass connected with the plate 16 will rise. Because the antibodies 22 are of weighted design, the weight increase of the plate 16 will be properly detectable.

When in the raw milk 8 at least substantially no progesterone bodies 20 are present and the antibodies 22 are supplied to the raw milk 8, these antibodies 22 will start to bind directly to the plate 16. The plate 16 will therefore be weighted immediately, which can be measured in a manner known per se.

A further advantage of the method according to Fig. 2 is that the regeneration of the biosensor can be carried out fast and simply. Because the antibodies 22 are weighted, they also dissociate readily from the bodies 18 again. When, therefore, the plate 16 is rinsed clean with, for instance, cleaning fluid, the antibodies 22 will easily detach, so that the biosensor can be used again. Because in the method according to Fig. 2 antibodies are added to the raw milk 8, the concentration of progesterone will preferably be determined on the basis of a separated sample of the raw milk.

It is also possible that the plate of the biosensor 2 is provided with a coating with progesterone antibodies 18, as shown in Fig. 3. The progesterone 20 which is present in the milk 8 will then bind to the antibodies 18'. The result is that the plate 16 will be weighted. The weighting of the plate 16, i.e., the extent to which weighting of the plate 16 occurs, is a measure for the concentration of progesterone in the raw milk 8. This weighting can be measured in a manner known per se. After the concentration of progesterone has been determined, the plate 16 can be rinsed clean in a manner known per se with, for instance, cleaning fluid for regeneration. The progesterone bodies 20 thereby detach and can be used for new detection of progesterone in raw milk 8.

In the method according to Fig. 3, it is theoretically possible that the biosensor 2 as described hereinbefore is included in the milk flow path of a milking robot.

To detect the weighting of the plate 16, the resonance frequency of the plate can be determined in a manner known per se. If the plate is provided with a metal oxide coating, the refractive index at the surface of the plate is found to be dependent on the amount of bodies (Fig. 3) or antibodies (Fig. 2) bound to the coating. This results in measurable changes in the reflection intensity of a laser beam with which the plate is illuminated. In this way too, through detection of the intensity of the reflected laser beam, the concentration can be determined. It is also possible that the amount of progesterone in the milk is determined but that the analysis of the measuring results does not take place until later. The obtained data can also be coupled with other data of the computer 4. It may also be important to consider the time at which the progesterone is detected in relation to other parameters that are dependent on this point in time. Thus, a pronouncement on fertility can be made on the ground of the determined amount of progesterone in combination with other parameters, such as activity and temperature of the animal.

## Claims

1. A method for determining the amount of progesterone in raw milk of cows, wherein a biosensor is contacted with the raw milk, while the amount of progesterone in the milk is determined with the aid of the biosensor, wherein use is made of
a) a biosensor having a plate which is provided with a coating of
of progesterone bodies and wherein antibodies to progesterone have been added to the raw milk; or
b) a biosensor having a plate which is provided with a coating of progesterone antibodies,
said method being **characterised in that** weighting of the plate is measured for determining the amount of progesterone.

2. A method according to claim 1, **characterised in that** the identity of the animal is determined, the amount of progesterone is determined in raw milk which has been respectively produced by the cow a plurality of times spread over time, while the identity of the animal, together with the determined amounts of progesterone and information about the times at which these amounts were determined and/or the milk was produced, are stored in a database.

3. A method according to claim 1 or 2, **characterised in that** the amount of progesterone in the milk is determined during milking.

4. A method according to claim 3, **characterised in that** the cow is milked with the aid of a milking plant/robot.

5. A method according to claim 4, **characterised in that** the biosensor is included in the milking plant/robot.

6. A method according to any of the preceding claims, **characterised in that** the amount of progesterone in a sample of the raw milk of the cow is determined.

7. A method according to any one of the preceding claims, **characterised in that** at least on the basis of the determined amount or amounts of progesterone it is determined whether a cow is in heat or in-calf or whether the cow has a defect of its reproductive apparatus.

8. A method according to any one of the preceding claims, **characterised in that** for about two weeks after a course of the determined amount of progesterone over time has reached a minimum, the progesterone content of the milk of the cow is not determined.

9. A method according to any one of the preceding claims, **characterised in that** if use is made of a biosensor having a surface provide with a coating with progesterone bodies, the antibodies are added to the milk are heavier than progesterone.

10. A method according to any one of the preceding claims, **characterised in that** the determined amount or amounts of progesterone is or are analysed at a later time than the time at which the sample is taken.

11. A method according to any one of the preceding claims, **characterised in that** the determined amount or amounts of progesterone is or are processed in combination with other data from a farm computer.

12. A method according to claim 7, **characterised in that** the determined amount or amounts of progesterone is or are processed in combination with other data, such as the time of determining the amount of progesterone, the activity and temperature of the animal, etc., for making a pronouncement about the fertility of the animal.

13. A method according to any one of the preceding claims, **characterised in that** the biosensor is connected with a computer for on-line determination of the amount of progesterone in the milk.

## Patentansprüche

1. Verfahren zur Bestimmung der Menge von Progesteron in Rohmilch von Kühen, wobei ein Biosensor mit der Rohmilch in Kontakt gebracht wird, während die Menge an Progesteron in der Milch mit Hilfe des Biosensors bestimmt wird, wobei folgendes verwendet wird:
a) ein Biosensor mit einer Platte, die mit einer Beschichtung aus Progesteronkörpern versehen ist, wobei der Rohmilch Antikörper gegen Progesteron zugesetzt wurden; oder
b) ein Biosensor mit einer Platte, die mit einer Beschichtung aus Progesteron-Antikörpern versehen ist;
wobei das Verfahren **dadurch gekennzeichnet ist, dass** das Gewicht der Platte gemessen wird; um die Menge an Progesteron zu bestimmen.

2. Verfahren gemäß Anspruch 1, **dadurch gekennzeichnet, dass** die Identität des Tiers bestimmt wird, die Menge an Progesteron in Rohmilch bestimmt wird, die von der Kuh jeweils mehrmals über die Zeit verteilt erzeugt wurde, während die Identität des Tiers zusammen mit den bestimmten Mengen an Progesteron und Informationen über die Zeitpunkte, zu denen diese Mengen bestimmt wurden und/oder die Milch erzeugt wurde, in einer Datenbank gespeichert wird.

3. Verfahren gemäß Anspruch 1 oder 2, **dadurch gekennzeichnet, dass** die Menge an Progesteron in der Milch während des Melkens bestimmt wird.

4. Verfahren gemäß Anspruch 3, **dadurch gekennzeichnet, dass** die Kuh mit Hilfe einer Melkanlage/eines Melkroboters gemolken wird.

5. Verfahren gemäß Anspruch 4, **dadurch gekennzeichnet, dass** der Biosensor in der Melkanlage/dem Melkroboter enthalten ist.

6. Verfahren gemäß einem der vorstehenden Ansprüche, **dadurch gekennzeichnet, dass** die Menge an Progesteron in einer Probe der Rohmilch von der Kuh bestimmt wird.

7. Verfahren gemäß einem der vorstehenden Ansprüche, **dadurch gekennzeichnet, dass** wenigstens auf der Basis der bestimmten Menge bzw. Mengen an Progesteron bestimmt wird, ob eine Kuh brünstig oder trächtig ist oder ob die Kuh einen Fehler ihres Reproduktionsapparats aufweist.

8. Verfahren gemäß einem der vorstehenden Ansprüche, **dadurch gekennzeichnet, dass** der Progesterongehalt der Milch von der Kuh etwa zwei Wochen lang, nachdem der zeitliche Verlauf der bestimmten Menge des Progesterons ein Minimum erreicht hat, nicht bestimmt wird.

9. Verfahren gemäß einem der vorstehenden Ansprüche, **dadurch gekennzeichnet, dass** ein Biosensor mit einer Oberfläche, die mit einer Beschichtung mit Progesteronkörpern versehen ist, verwendet wird und die zu der Milch gegebenen Antikörper schwerer als Progesteron sind.

10. Verfahren gemäß einem der vorstehenden Ansprüche, **dadurch gekennzeichnet, dass** die bestimmte Menge bzw. die bestimmten Mengen an Progesteron zu einem späteren Zeitpunkt analysiert werden als zu dem Zeitpunkt, an dem die Probe entnommen wird.

11. Verfahren gemäß einem der vorstehenden Ansprüche, **dadurch gekennzeichnet, dass** die bestimmte Menge bzw. die bestimmten Mengen an Progesteron in Kombination mit anderen Daten aus einem Farmcomputer verarbeitet werden.

12. Verfahren gemäß Anspruch 7, **dadurch gekennzeichnet, dass** die bestimmte Menge bzw. die bestimmten Mengen an Progesteron in Kombination mit anderen Daten, wie dem Zeitpunkt der Bestimmung der Menge an Progesteron, der Aktivität und Temperatur des Tiers usw., verarbeitet werden, um eine Aussage über die Fruchtbarkeit des Tiers zu treffen.

13. Verfahren gemäß einem der vorstehenden Ansprüche, **dadurch gekennzeichnet, dass** der Biosensor zur mitlaufenden Bestimmung der Menge des Progesterons in der Milch mit einem Computer verbunden ist.

## Revendications

1. Procédé pour déterminer la teneur en progestérone du lait cru des vaches, dans lequel on met le lait cru en contact avec un biodétecteur à l'aide duquel on détermine la teneur en progestérone du lait, et dans lequel on utilise soit a) un biodétecteur ayant une plaque qui est munie d'un revêtement de corps de progestérone, des anticorps envers la progestérone ayant été ajoutés au lait cru, soit b) un biodétecteur ayant une plaque qui est munie d'un revêtement d'anticorps envers la progestérone,
ledit procédé étant **caractérisé en ce que** l'on effectue la pesée de la plaque pour déterminer la teneur en progestérone.

2. Procédé selon la revendication 1, **caractérisé en ce que** l'animal est identifié et on détermine la teneur en progestérone du lait cru produit par la vache, à plusieurs reprises étalées dans le temps, l'identité de l'animal, les teneurs déterminées en progestérone et les informations concernant les moments où ces teneurs ont été déterminées et/ou le lait a été produit, étant stockées dans une base de données.

3. Procédé selon la revendication 1 ou 2, **caractérisé en ce que** la teneur en progestérone du lait est déterminée pendant la traite.

4. Procédé selon la revendication 3, **caractérisé en ce que** l'on trait la vache à l'aide d'une installation/robot de traite.

5. Procédé selon la revendication 4, **caractérisé en ce que** le biodétecteur est incorporé dans l'installation/robot de traite.

6. Procédé selon l'une quelconque des revendications précédentes, **caractérisé en ce que** l'on détermine la teneur en progestérone d'un échantillon du lait cru de la vache.

7. Procédé selon l'une quelconque des revendications précédentes, **caractérisé en ce que** l'on détermine, au moins en se basant sur la teneur déterminée ou les teneurs déterminées en progestérone, si une vache est en chaleur ou pleine ou si la vache présente un défaut de son appareil reproducteur.

8. Procédé selon l'une quelconque des revendications précédentes, **caractérisé en ce que**, pendant environ deux semaines après que la courbe de la teneur déterminée en progestérone en fonction du temps a atteint un minimum, on ne détermine pas la teneur en progestérone du lait de la vache.

9. Procédé selon l'une quelconque des revendications précédentes, **caractérisé en ce que**, si on utilise un biodétecteur ayant une surface munie d'un revêtement présentant des corps de progestérone, on ajoute au lait des anticorps qui sont plus lourds que la progestérone.

10. Procédé selon l'une quelconque des revendications précédentes, **caractérisé en ce que** l'on analyse la teneur déterminée ou les teneurs déterminées en progestérone à un moment postérieur au moment auquel l'échantillon est prélevé.

11. Procédé selon l'une quelconque des revendications précédentes, **caractérisé en ce que** l'on traite la teneur déterminée ou les teneurs déterminées en progestérone en combinaison avec d'autres données provenant d'un ordinateur de ferme.

12. Procédé selon la revendication 7, **caractérisé en ce que** l'on traite la teneur déterminée ou les teneurs déterminées en progestérone en combinaison avec d'autres données telles que le moment de la détermination de la teneur en progestérone, l'activité et la température de l'animal, etc., pour se prononcer sur la fertilité de l'animal.

13. Procédé selon l'une quelconque des revendications précédentes, **caractérisé en ce que** le biodétecteur est relié à un ordinateur pour la détermination en ligne de la teneur en progestérone du lait.
